(19) Europäisches Patentamt
European Patent Office
Office européen des brevets

(11) Veröffentlichungsnummer: **0 106 055**
**B1**

(12) # EUROPÄISCHE PATENTSCHRIFT

(45) Veröffentlichungstag der Patentschrift: **23.07.86**

(51) Int. Cl.⁴: **C 07 C 125/065**

(21) Anmeldenummer: **83107941.3**

(22) Anmeldetag: **11.08.83**

(54) **Verfahren zur Herstellung von Carbamaten.**

(30) Priorität: **08.09.82 DE 3233310**

(43) Veröffentlichungstag der Anmeldung: **25.04.84 Patentblatt 84/17**

(45) Bekanntmachung des Hinweises auf die Patenterteilung: **23.07.86 Patentblatt 86/30**

(84) Benannte Vertragsstaaten: **BE CH DE FR GB LI**

(56) Entgegenhaltungen: **ANGEWANDTE CHEMIE, Band 19, Nr. 9, September 1980, Seiten 659-748, WEINHEIM (DE), P. BALL et al.: "Carbonates and Polycarbonates from Urea and Alcohol", Seiten 718-720**

(73) Patentinhaber: **BASF Aktiengesellschaft, Carl-Bosch- Strasse 38, D-6700 Ludwigshafen (DE)**

(72) Erfinder: **Merger, Franz, Dr., Max- Slevogt- Strasse 25, D-6710 Frankenthal (DE)**
Erfinder: **Nestler, Gerhard, Dr., Van- Leyden-Strasse 17, D-6700 Ludwigshafen (DE)**
Erfinder: **Towae, Friedrich, Dr., Parkstrasse 22, D-6700 Ludwigshafen (DE)**

**EP 0 106 055 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die Erfindung betrifft ein Verfahren zur Herstellung von N- und O-substituierten Carbamaten durch Umsetzung von Harnstoff mit Aminopropanolen bei einer Temperatur von mindestens 140°C.

Aus Houben-Weyl, Methoden der Organischen Chemie, Band 8, Seiten 140—141, ist bekannt, daß man Harnstoff mit Alkoholen zu O-substituierten Carbamaten umsetzen kann. Eine Alkylierung des Harnstoffs durch den entsprechenden Alkohol zu N-substituierten Harnstoffen wird hierbei nicht beobachtet. Die Umsetzung erfolgt drucklos am Siedepunkt des Gemisches, gegebenenfalls mit Zinkacetat oder Stannichlorid als Katalysator. Nach Paquin (Z. f. Naturforschung *1*, Seiten 518—523) kann durch Zusatz von Schwermetallsalzen die Ausbeute gesteigert werden. Der Nachteil dieser Methoden besteht jedoch darin, daß verschiedene Nebenprodukte, z.B. Biuret, Allophansäureester, Cyansäure bzw. Carbonate, in beträchtlichen Mengen entstehen und die Ausbeute entsprechend schlecht ist (DE—OS 24 59 765).

Um die Bildung dieser Nebenprodukte zu verhindern, wird in der DE—OS 16 43 635 die Verwendung von Nickelsalzen als Katalysatoren vorgeschlagen. Es lassen sich nach dieser Methode zwar Ausbeuten von 94% erzielen, doch lange Reaktionszeiten und die abwasserbelastende Entfernung der cancerogenen Nickelsalze aus dem Reaktionsgemisch bzw. die Aufarbeitung der entsprechenden Abwässer haben dazu geführt, daß dieses Verfahren keine wirtschaftliche Bedeutung erlangt hat. In der DE—OS 24 59 765 wird nun die Verwendung von Nickel enthaltenden Ionenaustauschern als Katalysatoren vorgeschlagen. Nachteilig bei diesem Verfahren ist jedoch wieder das Arbeiten mit cancerogenen Nickelsalzen, die aufwendige und abwasserbelastende Herstellung des Katalysators (DE—OS 24 59 765 Beispiel 1a), die Bildung von Carbonaten und die im Reaktionsgemisch gelösten Nickelverbindungen, die aufgrund ihrer Toxizität entfernt werden müssen.

Aus DE—OS 30 21 554 ist ein Verfahren zur Herstellung von Carbonaten durch Umsetzung von Carbamidsäureestern mit Alkoholen bei einer Temperatur oberhalb 140°C bekannt, wobei man während der Umsetzung den gebildeten Ammoniak abtrennt. Vorteilhaft führt man die Umsetzung in Gegenwart von tertiären Aminen oder Amidinen als Katalysatoren oder in Gegenwart von Verbindungen der Metalle unter den Elementen der Gruppen Ia, Ib, IIa, IIb, IIIa, IIIb, IVa, IVb, Va, Vb, VIb, VIIb, VIIIb des Periodischen Systems durch. Wie Beschreibung und Beispiele zeigen, werden keine Aminoalkanole verwendet.

Es wurde nun gefunden, daß man N- and O-substituierte Carbamate der Formel

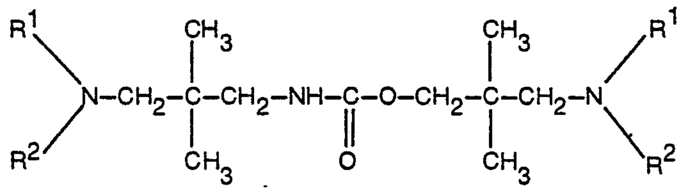

I,

in der die einzelnen Reste $R^1$ und $R^2$ gleich oder verschieden sein können und jeweils einen aliphatischen, cycloaliphatischen oder araliphatischen Rest bedeuten, $R^1$ und $R^2$ zusammen mit dem benachbarten Stickstoffatom auch einen heterocyclischen Ring bezeichnen können, durch Umsetzung von Harnstoff mit Alkoholen vorteilhaft erhält, wenn man Harnstoff mit Aminopropanolen der Formel

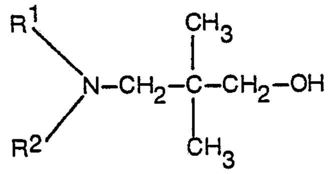

II

in der die Reste $R^1$ und $R^2$ vorgenannte Bedeutung besitzen, bei einer Temperatur von mindestens 140°C umsetzt.

Die Umsetzung kann für den Fall der Verwendung von 3-Dimethylamino-2,2-dimethylpropanol-(1) durch die folgenden Formeln wiedergegeben werden:

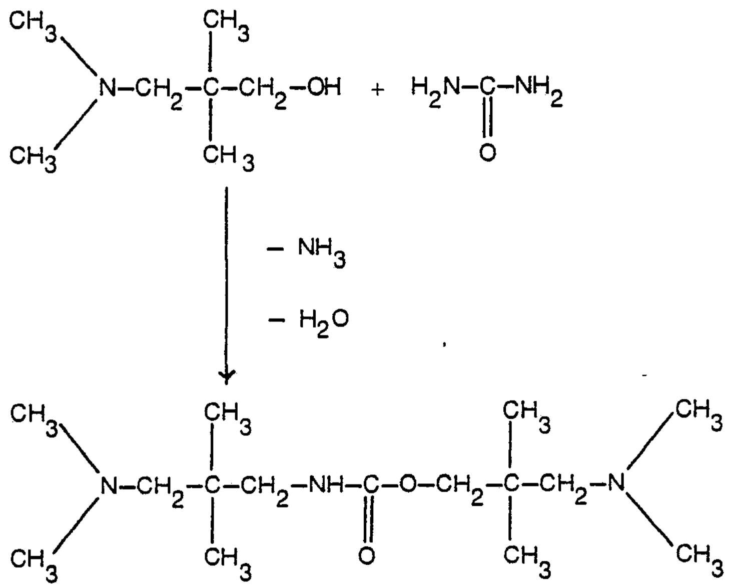

Im Vergleich zu den bekannten Verfahren liefert das Verfahren nach der Erfindung auf einfacherem und wirtschaftlicherem Wege gleichzeitig am Stickstoffatom und am Sauerstoffatom substituierte Carbamate in guter Ausbeute, Raum-Zeit-Ausbeute und Reinheit. Es war im Hinblick auf vorgenannte Veröffentlichungen überraschend, daß das Verfahren nach der Erfindung ohne Verwendung von Katalysatoren gleichzeitig am Stickstoffatom und am Sauerstoffatom substituierte Carbamate,auf einfachem Wege in guter Ausbeute und Reinheit ohne wesentliche Bildung von Nebenprodukten liefert. Man hätte insbesondere mit Bezug auf die deutsche Offenlegungsschrift 16 43 635 zumindest keine guten Ausbeuten und keine hohe Reinheit und sogar eine Verschlechterung der Ergebnisse erwarten müssen, da, entgegen der Lehre der Offenlegungsschrift, nur Nickelsalze und diese in einer Menge von 0,1 bis 5 Gew.%, bezogen auf Harnstoff, zu verwenden, und entgegen der Lehre der DE—OS 24 59 765 Kationenaustauscher und das als Kation an den Austauscher gebundenen Nickel zu verwenden, das erfindungsgemäße Verfahren ohne Katalysatoren durchgeführt wird. Durch den Wegfall von Katalysatoren und Austauschern ist das erfindungsgemäße Verfahren betriebssicherer, vermeidet toxische Produkte und Abwasserprobleme und ist somit umweltfreundlicher.

Im Hinblick auf die DE—OS 30 21 554 ist es überraschend, daß Aminopropanole, die in 2-Stellung zwei Methylgruppen tragen, mit ihrer speziellen Struktur nicht Carbonate sondern Carbamate liefern; ebenso konnte man den vorgenannten Veröffentlichungen nicht entnehmen, daß die erfindungsgemäß strukturierten Aminopropanole zu Carbamaten, die gleichzeitig am Stickstoff- und am Sauerstoff substituiert sind, umgesetzt werden.

Harnstoff kann mit den Ausgangsstoffen II in stöchiometrischer Menge, im Überschuß oder im Unterschuß, vorzugsweise in einer Menge von 0,5 bis 10, insbesondere 0,8 bis 2 Mol Ausgangsstoff II je Mol Harnstoff umgesetzt werden; vorgenannte Mengen werden in der Regel bei Verwendung von zusätzlichen Lösungsmitteln verwendet. Arbeitet man in Abwesenheit zusätzlicher Lösungsmittel, d.h. dienen die Reaktionspartner selbst als Reaktionsmedium, so sind Mengen von 0,8 bis 10, insbesondere 1 bis 5 Mol Ausgangsstoff II je Mol Harnstoff vorteilhaft.

Bevorzugte Ausgangsstoffe II und dementsprechend bevorzugte Endstoffe I sind solche, in deren Formeln die einzelnen Reste $R^1$ und $R^2$ gleich oder verschieden sein können und jeweils einen Alkylrest mit 1 bis 18, vorzugsweise 1 bis 8, insbesondere 1 bis 6 Kohlenstoffatomen, einen Alkenylrest mit 2—12, zweckmäßig 2—6 Kohlenstoffatomen, einen Cycloalkylrest mit 3 bis 8 Kohlenstoffatomen oder einen Aralkylrest mit 7 bis 12 Kohlenstoffatomen bedeuten, $R^1$ und $R^2$ zusammen mit dem benachbarten Stickstoffatom auch einen 3- bis 7-gliedrigen Ring, der noch ein weiteres Stickstoffatom oder ein Sauerstoffatom oder ein Schwefelatom enthalten kann, bezeichnen können. Die vorgenannten Reste können noch durch unter den Reaktionsbedingungen inerte Gruppen, z.B. Alkylgruppen oder Alkoxygruppen mit jeweils 1 bis 4 Kohlenstoffatomen, substituiert sein.

Es kommen z.B. die folgenden Aminopropanole als Ausgangsstoffe II in Frage: 2,2-Dimethyl-3-di-benzyl-, 2,2-Dimethyl-3-di-methyl-, 2,2-Dimethyl-3-di-isopropyl-, 2,2-Dimethyl-3-di-ethyl-, 2,2-Dimethyl-3-di-sek.-Butyl-, 2,2-Dimethyl-3-di-cyclohexyl-, 2,2-Dimethyl-3-di-pentyl-, 2,2-Dimethyl-3-di-cyclopentyl-, 2,2-Dimethyl-3-di-phenylethyl-, 2,2-Dimethyl-3-di-propyl-, 2,2-Dimethyl-3-di-decyl-, 2,2-Dimethyl-3-di-n-octyl-, 2,2-Dimethyl-3-di-tert-butyl-, 2,2-Dimethyl-3-di-isobutyl-, 2,2-Dimethyl-3-di-n-butyl-aminopropanol-(1), N-Methyl-N-phenyl-2,2-dimethyl-3-aminopropanol-(1), 3-Piperidino-, 3-Morpholino-, 3-Pyrrolidino-, 3-Aziridino-, 3-Thiomorpholino-2,2-dimethylpropanol-(1); bevorzugt 3-Dimethylamino-2,2-dimethylpropanol-(1), 3-Dipropylamino-2,2-dimethylpropanol-(1), 3-Diethylamino-2,2-dimethylpropanol-(1), 3-(N-Ethyl-N-methyl)-amino-2-dimethyl-propanol-(1), 3-Piperidino-2,2-dimethylpropanol-(1), 3-Morpholino-2,2-dimethylpropanol-(1), 3-Pyrrolidino-2,2-dimethylpropanol-(1).

Die Reaktion wird bei einer Temperatur von mindestens 140°C zweckmäßig zwischen 140 und 260°C vorzugsweise von 160 bis 240°C, drucklos, unter Überdruck oder Unterdruck, kontinuierlich oder diskontinuierlich durchgeführt. Im allgemeinen dient der Ausgangsstoff II gleichzeitig auch als Medium der Reaktion, gegebenenfalls kommen auch unter den Reaktionsbedingungen inerte, organische Lösungsmittel in Frage, z.B. aromatische Kohlenwasserstoffe wie Benzol, Toluol, Ethylbenzol, o-, m-, p-Xylol, Isopropylbenzol und entsprechende Gemische. Zweckmäßig verwendet man das Lösungsmittel in einer Menge von 50 bis 1000 Gew.%, vorzugsweise von 100 bis 500 Gew.% bezogen auf Ausgangsstoff II. Während der Umsetzung wird im allgemeinen ein pH von 7 und mehr als 7 eingehalten.

Die Reaktion kann wie folgt durchgeführt werden: Ein Gemisch von Harnstoff und Ausgangsstoff II wird während 2 bis 10 Stunden bei der Reaktionstemperatur gehalten. Der bei der Reaktion entstehende Ammoniak kann durch Destillation aus der Reaktionslösung entfernt werden. Teilweise bildet sich Ammoniumcarbaminat, das in einem nachgeschalteten Kühler niedergeschlagen und zur Harnstoffsynthese zurückgeführt werden kann. Dann wird das Reaktionsgemisch abgekühlt und filtriert. Aus dem Filtrat wird in üblicher Weise, z.B. durch Destillation, der Endstoff abgetrennt.

Die nach dem Verfahren der Erfindung herstellbaren Verbindungen sind wertvolle Ausgangsstoffe bei der Herstellung von Textilausrüstungsmitteln, Stabilisatoren, Weichmachern, Farbstoffen und Pflanzenschutzmitteln. Durch thermische Spaltung können Isocyanate hergestellt werden. Bezüglich der Verwendung wird auf die vorgenannten Veröffentlichungen verwiesen.

### Beispiel 1

In einer Rührapparatur mit aufgesetztem, auf 80°C gehaltenen Kühler wurden 360 g Harnstoff mit 1965 g 3-Dimethylamino-2,2-dimethylpropanol-(1) 7,5 Stunden lang auf 180°C erhitzt. Nach beendeter Reaktion ließ man abkühlen und destillierte 1025 g 3-Dimethylamino-2,2-dimethylpropanol-(1) ab. Es verblieben 770 g (74,8% der Theorie) N-(3-Dimethylamino-2,2-dimethylpropyl)-O-(3'-dimethylamino-2',2'-dimethylpropyl)-carbamat vom Siedepunkt 116—117°C/ 0,5 mbar.

### Beispiele 2—6

Es wurde gemäß Beispiel 1 verfahren, wobei andere Aminopropanole II in demselben Molverhältnis mit Harnstoff bei verschiedenen Temperaturen und mit unterschiedlichen Reaktionszeiten eingesetzt wurden. Die Ergebnisse sind in der Tabelle aufgeführt.

TABELLE

| Bsp. Nr. | $R^1$, $R^2$ | Zeit in h | Temperatur in °C | Endstoff I (bezogen auf umgesetzten Aminopropanol II) in % der Theorie |
|---|---|---|---|---|
| 2 | 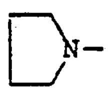 | 6 | 180 | 81.3 |
| 3 | 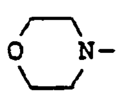 | 10 | 230 | 53.7 |
| 4 | 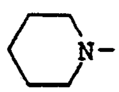 | 7 | 210 | 69.0 |
| 5 | $—C_2H_5$, $—C_2H_5$ | 7 | 215 | 73.4 |
| 6 | $C_3H_7—$, $C_3H_7—$ | 7 | 210 | 74.8 |

**Patentanspruch**

Verfahren zur Herstellung von N- und O-substituierten Carbamaten der Formel

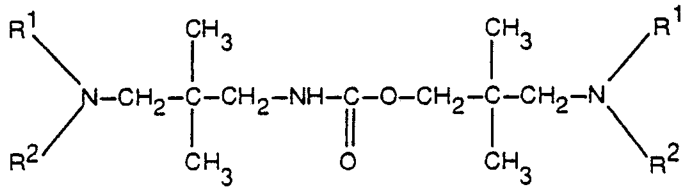

I,

in der die einzelnen Reste $R^1$ und $R^2$ gleich oder verschieden sein können und jeweils einen aliphatischen, cycloaliphatischen oder araliphatischen Rest bedeuten, $R^1$ und $R^2$ zusammen mit dem benachbarten Stickstoffatom auch einen heterocyclischen Ring bezeichnen können, durch Umsetzung von Harnstoff mit Alkoholen, dadurch gekennzeichnet, daß man Harnstoff mit Aminopropanolen der Formel

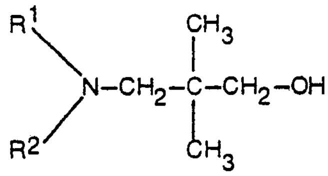

II

in der die Reste $R^1$ und $R^2$ die vorgenannte Bedeutung besitzen, bei einer Temperatur von mindestens 140°C umsetzt.

**Revendication**

Procédé de préparation de carbamates N- et O-substitués de la formule

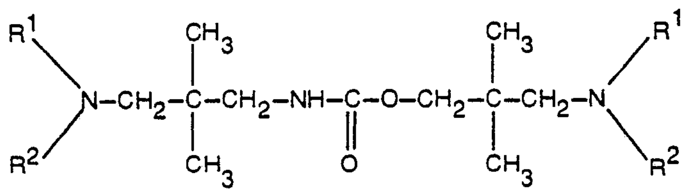

I,

dans laquelle les divers substituants $R^1$ et $R^2$ peuvent être identiques ou différents et désignent chacun un groupe aliphatique, cycloaliphatique ou araliphatique, $R^1$ et $R^2$ pouvant aussi former avec l'atome d'azote adjacent un noyau hétérocyclique, par réaction d'urée et d'alcools, caractérisé en ce que l'on fait réagir de l'urée avec des aminopropanols de la formule

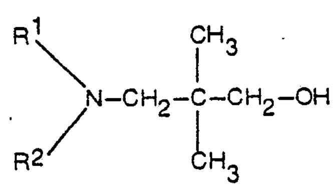

II

dans laquelle $R^1$ et $R^2$ possèdent la signification définie, à une température au moins égale à 140°C.

**Claim**

A process for the preparation of an N- and O-substituted carbamate of the formula

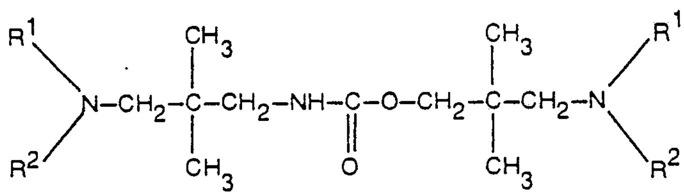

I,

where the individual radicals $R^1$ and $R^2$ are identical or different and are each an aliphatic, cycloaliphatic or

araliphatic radical, or $R^1$ and $R^2$, together with the adjacent nitrogen atom, can furthermore form a heterocyclic ring, by reacting urea with an alcohol, wherein urea is reacted with an aminopropanol of the formula

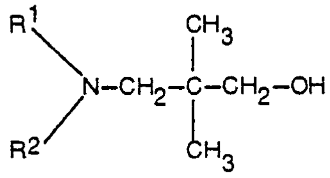

II .

where $R^1$ and $R^2$ have the above meanings, at a temperature of at least 140°C.